# EUROPEAN PATENT SPECIFICATION

(11) **EP 2 231 048 B1**
(45) Date of publication and mention of the grant of the patent: **22.06.2016**
(21) Application number: 08866358.8
(22) Date of filing: 12.12.2008
(51) Int. Cl.: A61B 18/02

(54) **SYSTEM FOR CONTROLLABLY DELIVERING LIQUID COOLANT TO A CRYO-ABLATION DEVICE**
SYSTEM ZUR GESTEUERTEN ABGABE EINER KÜHLFLÜSSIGKEIT AN EIN KRYOABLATIONSGERÄT
SYSTÈMES DE DISTRIBUTION CONTRÔLÉE DE RÉFRIGÉRANT LIQUIDE À UN DISPOSITIF DE CRYOABLATION

(30) Priority: 27.12.2007 US 17131 P
(43) Date of publication of application: 29.09.2010
(73) Proprietor: Boston Scientific Scimed, Inc., Maple Grove, MN 55311-1566 (US)
(72) Inventor: HON, Raphael, Irvine California 92614 (US)
(74) Representative: Pfenning, Meinig & Partner mbB
(86) International application number: PCT/US2008/086620
(87) International publication number: WO 2009/085666

(56) References cited:
- EP-A- 1 514 529
- WO-A-98/52479
- WO-A-2007/076123
- US-A1- 2001 021 847

## Description

### FIELD OF INVENTION

The invention generally relate to cryo-ablation devices and, more particularly, to systems and methods for delivering liquid coolant to a cryo-ablation device.

### BACKGROUND

Atrial fibrillation is a condition in which upper chambers of the heart beat rapidly and irregularly. One known manner of treating atrial fibrillation is to administer drugs in order to maintain normal sinus rhythm and/or to decrease ventricular rhythm. Known drug treatments, however, may not be sufficiently effective, and additional measures such as cardiac tissue ablation must often be taken to control the arrhythmia.

Known ablation procedures for treating atrial fibrillation include performing transmural ablation of the heart wall or adjacent tissue walls using radio frequency (RF) energy. One known ablation procedure is referred to as transmural ablation and involves burning or ablating cardiac tissue and forming lesions to break up circuits believed to drive atrial fibrillation. Transmural ablation may be grouped into two main categories of procedures: endocardial ablation and epicardial ablation. Endocardial procedures are performed from inside the wall (typically the myocardium) that is to be ablated. Endocardial ablation is generally carried out by delivering one or more ablation devices into the chambers of the heart by catheter delivery, typically through the arteries and/or veins of the patient. In contrast, epicardial procedures are performed from the outside wall (typically the myocardium) of the tissue that is to be ablated. Epicardial procedures are often performed using devices that are introduced through the chest and between the pericardium and the tissue to be ablated.

While RF transmural ablation has been used effectively in the past, cryogenic ablation has received increased attention for treatment of atrial fibrillation in view of the effectiveness of cryo-ablation procedures with fewer side effects. One known endocardial cryo-ablation procedure involves inserting a catheter into the heart, e.g., through the leg of a patient. Once properly positioned, a portion of the catheter, typically the tip of the catheter, is cooled to a sufficiently low temperature by use of a liquid coolant or refrigerant such as nitrous oxide, e.g., to sub-zero temperatures of about -75°C., in order to freeze tissue believed to conduct signals that cause atrial fibrillation. The frozen tissue eventually dies so that the ablated tissue no longer conducts electrical impulses that are believed to cause or conduct atrial fibrillation signals.

One known catheter-based cryo-ablation system includes a refrigerant source such as a compressed gas bottle or tank containing nitrous oxide, the intended use of which is to release or draw gaseous nitrous oxide. The nitrous oxide in the tank may be saturated such that the tank includes nitrous oxide in gaseous form as well as nitrous oxide in liquid form. Certain known systems are configured to extract the liquid portion of saturated nitrous oxide from the tank, e.g., by inverting the tank such that liquid nitrous oxide flows within a portion of the tank to allow the liquid nitrous oxide to be withdrawn from the tank. The extracted liquid nitrous oxide is then injected directly into the catheter by the tank pressure. Other known devices attempt to address the difficulties and inconvenience associated with inverting a refrigerant tank by utilizing a siphon tube. Known systems also use a cooling element that condenses gaseous nitrous oxide released from the tank into a liquid, and liquid nitrous oxide is delivered to or injected directly into a catheter.

Known systems, however, have a number of shortcomings. For example, it is inconvenient to have to invert gas tanks in order to extract liquid nitrous oxide from a supply tank, which is intended to deliver gaseous refrigerant rather than liquid refrigerant. Further, known systems provide only limited control over the delivery of liquid nitrous oxide that is extracted from a supply tank generated by a cooling element and delivered to the catheter due to their reliance on the pressure of the supply tank to deliver liquid nitrous oxide to the cryo-ablation catheter. Consequently, low supply tank pressures may reduce the performance and effectiveness of the cryo-ablation device, and these negative effects may be more noticeable as the nitrous oxide in supply tank is depleted and the tank pressure is reduced over time. Further, if the supply tank valve is closed, nitrous oxide liquid remains within the supply line downstream of the supply tank. Consequently, remaining pressure in the supply line continues to push nitrous oxide liquid through the system to the cryo-ablation catheter despite the desire of the clinician to stop the flow of flow of coolant. Additionally, the pressure of the liquid nitrous oxide at the cryo-ablation catheter is governed and limited by the pressure of the tank, which pushes the extracted liquid nitrous oxide to the cryo-ablation catheter. Such pressures may be difficult to control and may be insufficient due to limitations associated with the internal tank pressure. Thus, if reduced liquid pressure at the catheter is desired, the pressure of the supply tank or bottle can be reduced, but it may not be able to increase the liquid pressure at the catheter above the pressure of the supply tank in known systems. These issues limit the capabilities and effectiveness of a cryo-ablation system in various ways.

For example, there may be instances when higher liquid nitrous oxide pressures are required to overcome the pressure of a cryo-ablation catheter such that liquid nitrous oxide can be injected or forced into the cryo-ablation catheter. Certain high resistance catheters, therefore, may not be compatible with systems that rely on tank pressures, which may not be high enough to overcome catheter pressure or resistance. As a further example, the temperature of the liquid nitrous oxide may increase as it flows through system supply lines and the catheter, thereby causing the liquid nitrous oxide to vaporize, resulting in less effective cryo-ablation. One method to maintain the nitrous oxide in liquid form at elevated temperatures is to increase the pressure of the liquid. However, with known systems that rely on the pressure of the tank, the ability to increase the pressure of the nitrous oxide liquid may be limited or not possible. Further, having to heat the supply tank to increase pressure is inconvenient, requires additional components, adds one more parameters to monitor and control, and increases the temperature of the nitrous oxide, which is generally not desirable for purposes of cryo-ablation.

US 2001/0021847 discloses a system comprising a liquid or close to liquid gaseous supply of coolant that is cooled with a subcooler to the liquid state led through a catheter by using the force of a vacuum pump that expels the liquid coolant into a tank.

### SUMMARY

The invention is defined by appended claim 1, preferred embodiments are described in the dependent claims.

In accordance with one embodiment, a system for delivering a liquid cooiant to a cryo-ablation device that is used for cryogenically ablating tissue includes a coolant supply, a cooling element such as a heat exchanger and an actuator. The cooling element is configured to liquefy gaseous coolant that is released from the coolant supply. The actuator is in fluid communication with the cooling element and the cryo-ablation device and defines a chamber. The actuator is configured to controllably draw liquid coolant into the chamber and controllably expel liquid coolant from the chamber for delivery to the cryo-ablation device.

In accordance with another embodiment, a system for delivering liquid nitrous oxide to a cryo-ablation device that is used to cryogenically ablate tissue includes a tank for storing gaseous nitrous oxide, a cooling element, a container or reservoir and an actuator. The cooling element is configured to liquefy gaseous nitrous oxide that is released from the tank. Liquefied nitrous oxide is stored in the container. The actuator is in fluid communication with the container and the cryo-ablation device and defines a chamber. The actuator is configured to controllably draw liquid nitrous oxide from the container and into the chamber, and to controllably expel liquid nitrous oxide from the chamber for delivery to the cryo-ablation device.

Also disclosed is a method of delivering a liquid coolant to a cryo-ablation device that is used to cryogenically ablate tissue. The method includes releasing gaseous coolant from a coolant supply and liquefying gaseous coolant with a cooling element. The method further includes controllably drawing liquid coolant into an actuator chamber in fluid communication with the cooling element and the cryo-ablation device, controllably expelling liquid coolant from the chamber and delivering expelled liquid coolant to the cryo-ablation device.

Further, in one or more embodiments, one-way valves may be associated with an input and an output of the actuator so that liquid coolant flows in one direction and enters the chamber of the actuator, and also flows in one direction when the liquid coolant is expelled or forced out of the chamber.

The actuator can be a syringe, a piston or a pump that is operable to change the size of the chamber and draw cooling fluid in and push or expel cooling fluid out from the chamber. For example, a syringe actuator may include a hollow barrel that defines the chamber, a gasket and a plunger. The plunger is controllably movable to displace the gasket and to expand the chamber to controllably draw liquid coolant into the chamber, and to contract the chamber to controllably expel liquid coolant from the chamber. The actuator can be configured so that a pressure of liquid coolant within the chamber is greater than a pressure of gaseous coolant within the coolant supply. For example, the pressure of the liquid coolant within the chamber is about 100 psi to about 1,500 psi, and is greater than the pressure of the gaseous coolant within the coolant supply. In this manner, liquid coolant can advantageously be controllably delivered to a cryo-ablation device and at higher pressures than supplies or tanks that store a coolant in gaseous form.

In one embodiment, the cooling element and the actuator are contained within a common cooling environment and, if necessary, the cooling element can be contained within a separate cooling environment within the common cooling environment to provide cooled environments with desired or different temperature profiles.

In one or more embodiments, the cryo-ablation device can be a cryo-ablation catheter and may be utilized for cryogenically ablating various types of tissue including endocardial tissue.

### BRIEF DESCRIPTION OF THE DRAWINGS

Embodiments will be described and explained with additional specificity and detail with reference to the accompanying drawings in which:
Fig. 1 illustrates a system constructed according to one embodiment for controllably delivering liquid coolant to a cryo-ablation device;
Fig. 2 is a flow chart of a method of controllably delivering liquid coolant to a cryo-ablation device according to one embodiment using the system shown in Fig. 1;
Fig. 3 illustrates a system constructed according to one embodiment that includes a heat exchanger cooling element, a container and an actuator that controllably draws liquid coolant into a chamber and controllably forces cooling liquid from the chamber for delivery to a cryo-ablation device;
Fig. 4 further illustrates one embodiment of an actuator shown in Fig. 3;
Fig. 5 further illustrates the actuator embodiment shown in Fig. 4 and liquid coolant flowing through a first one-way valve to fill a chamber;
Fig. 6 further illustrates the actuator shown in Fig. 4 and further filling of the chamber with liquid coolant by pulling back a component of the actuator to enlarge the size of the chamber;
Fig. 7 further illustrates the actuator shown in Fig. 4 and forcing or expelling liquid coolant out of the chamber by pushing forward a component of the actuator to reduce the size of the chamber;
Fig. 8 shows the system shown in Fig. 3 and temperature and pressure parameters within the system;
Fig. 9 illustrates a system constructed according to another embodiment that includes components as shown in Fig. 3 and a bypass valve connected between the cooling element and container;
Fig. 10 illustrates a system not constructed according to the invention that does not include a bypass valve or container for storing liquid coolant; and
Fig. 11 is a flow chart illustrating controlled delivery of liquid coolant to a cryo-ablation device using the system shown in Fig. 10.

### DETAILED DESCRIPTION OF ILLUSTRATED EMBODIMENTS

Embodiments provide liquid coolant or refrigerant delivery systems that provide one or more intermediate components including a controllable actuator configured to control liquid coolant that is delivered to a cryo-ablation device. With embodiments, liquid coolant is not provided directly from a cooling element to a cryo-ablation device. Instead, embodiments utilize an indirect system that includes an intermediate actuator, which allows a clinician to control the timing, quantity and/or pressure of liquid coolant that is delivered to the cryo-ablation device.

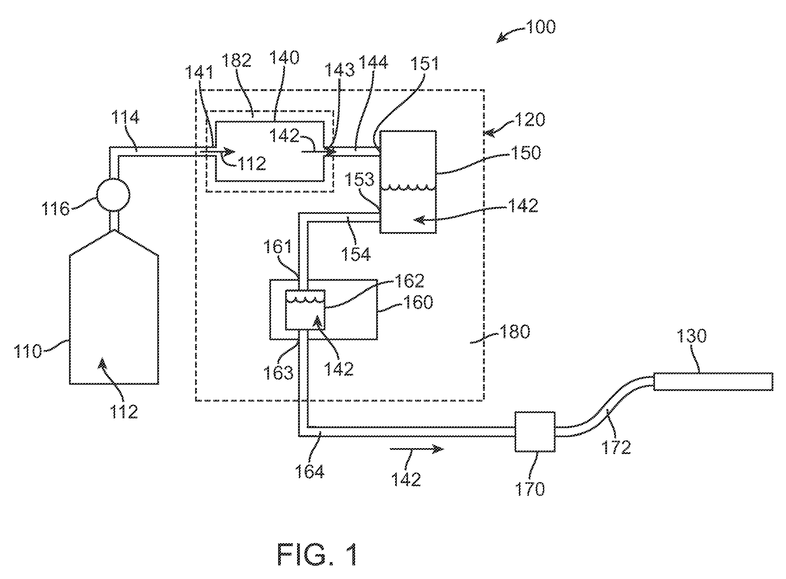

With this indirect or intermediate configuration, embodiments can provide liquid coolant to cryo-ablation devices in a controlled and precise manner at various pressures independent of the pressure of a supply tank. For example, with embodiments, the pressure of the liquid coolant delivered to the cryo-ablation device may be less than, the same as, or greater than the pressure of a supply tank that provides coolant in gaseous form, thereby providing flexibility and control over liquid coolant pressures and delivery. These capabilities are particularly useful when utilizing higher pressure catheters and when it is necessary to maintain coolant in liquid form when the coolant is at elevated temperatures. Embodiments achieve these capabilities without having to heat coolant supply tanks, invert coolant supply tanks or use siphon tubes, which are used in certain known systems. Further aspects of embodiments are described with reference to Figs. 1-11.

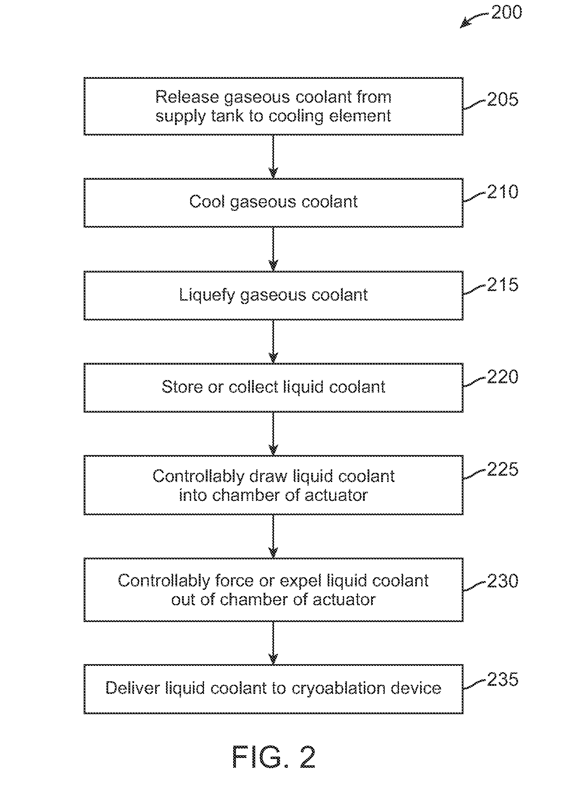

Referring to Fig. 1, and with further reference to Fig. 2, a system 100 constructed according to one embodiment for controllably delivering coolant or refrigerant, such as a flowable liquid coolant (generally referred to as "liquid coolant") to a cryo-ablation device 130 includes a supply tank or bottle 110 (generally referred to as a "supply tank") that includes coolant 112 in gaseous form and a control system 120. The control system 120 is configured to controllably deliver liquid coolant to the cryo-ablation device 130 by controlling the timing, quantity and/or pressure of the liquid coolant delivered to the cryo-ablation device 130.

In the illustrated embodiment, the control system 120 includes a cooling element 140 having an inlet 141 and an outlet 143. The cooling element inlet 141 is in fluid communication with the supply tank 110 through a suitable gas line 114 and one or more valves 116 that are controlled to release gaseous coolant 112 from the supply tank 110 to the cooling element 140 at stage 205. Gaseous coolant 112 released from the supply tank 110 enters the cooling element 140 through a cooling element inlet 141, cools the gaseous coolant 112 at stage 205, and condenses the gaseous coolant 112 into a liquid at stage 210. In the illustrated embodiment, liquid coolant 142 exits the cooling element 140 through the outlet 143 and enters an inlet 151 of a container, reservoir or storage vessel 150 (generally referred to as "container"), which stores or collects liquid coolant 142 generated by the cooling element 140 at stage 215.

It should be understood that embodiments can be implemented using various gaseous and liquid refrigerants or coolants 112, 142, and that embodiments may be utilized in connection with various types of cryo-ablation devices 130 for use in various cryo-ablation surgical procedures and treatments. For example, the supply tank 110 may store N₂0, C0₂, N₂ gaseous coolants 112, and the resulting liquid coolant 142 delivered to the cryo-ablation device 130 may also be N₂0, C0₂, N₂ and other liquid coolants 142 that are suitable for use in cryo-ablation procedures. Embodiments may be utilized to controllably deliver liquid coolant 142 to cryo-ablation devices 130 including cryo-ablation catheters (e.g., balloon or point type catheters) and other suitable cryo-ablation devices 130 for purposes of cryogenically ablating cardiac tissue including endocardial tissue, e.g., for treatment of atrial fibrillation, flutter and other cardiac conditions. Further, embodiments may be used with cryo-ablation devices 130 that are used in other procedures and treatments including, but not limited to, cryo-ablation of cancerous tissue and treatment of skin disorders that are typically treated using other sources of ablation energy such as a laser. For ease of explanation, reference is made to a supply tank 110 that stores a gaseous N₂0 or nitrous oxide 112, and a control system 120 that controllably delivers liquid nitrous oxide 142 to a cryo-ablation catheter 130 for use in ablating endocardial tissue to treat atrial fibrillation. Thus, it should be understood that embodiments may be used with various gaseous and liquid coolants 112, 142, various cryo-ablation devices 130, and in various cryo-ablation procedures and treatments.

An outlet 153 of the container 150 is in fluid communication with the cooling element 140 through a supply line 144 and, if necessary, one or more valves (not shown in Fig. 1). The container 150 is also in fluid communication with a controller or actuator 160 (generally referred to as "actuator") through a suitable supply line 154 and, if necessary, one or more valves (not shown in Fig. 1). In the illustrated embodiment, the container 150 is connected between the cooling element 140 and the actuator 160, and the actuator 160 is connected between the container 150 and the cryo-ablation catheter 130.

The actuator 160 defines an internal chamber, cavity or reservoir 162 (generally referred to as "chamber"). Liquid coolant 142 provided or released by the container 150 at stage 220 is controllably drawn into the chamber 162 through an inlet 161 at stage 225, and controllably pushed, forced or expelled from the chamber 162 through an outlet 163 at stage 230. Liquid coolant 142 that is controllably expelled from the chamber 162 may be provided to a console or interface 170 of the cryo-ablation catheter 130 through suitable connectors or supply lines 172 for use in a cryo-ablation procedure at stage 235.

According to one embodiment, the actuator 160 is in the form of a pump, piston, syringe or similar device. For example, a syringe actuator may include a hollow barrel that defines an internal chamber 162, a gasket disposed inside the hollow barrel within the hollow barrel and that engages an inner surface of the barrel and that forms a seal with the inner surface of the barrel, and a plunger. The plunger is controllably movable to displace the gasket, thereby expanding the size of the chamber 162 to controllably draw liquid coolant 142 into the chamber 162, and to controllably contract or reduce the size of the chamber 162 to controllably expel or force liquid coolant 142 from the chamber 162.

In the illustrated embodiment, the cooling element 140, the container 150 and the actuator 160 are located in a common, cooled environment 180, which may be maintained at temperatures of about -75°C. to about 15°C. Other system 120 configurations may be utilized as necessary and depending on the temperatures at which certain components must be maintained. For example, the container 150 may be located within the common environment 180 or within a separate external cooled environment (not shown), which is maintained at a temperature of about -75°C. to about 15°C. As a further example, the cooling element 140 may be contained within a separate cooled environment 182 within the common cooled environment 180 (as shown in Fig. 1) or outside of the common cooled environment 180. The cooling environment 182 may be maintained at a temperature of about -40°C. to about 15°C. For ease of explanation, control system 120 configurations are described with reference to the cooling element 140, container 150 and actuator 160 components being contained within a cooled environment 180, but different temperatures and environments may also be utilized.

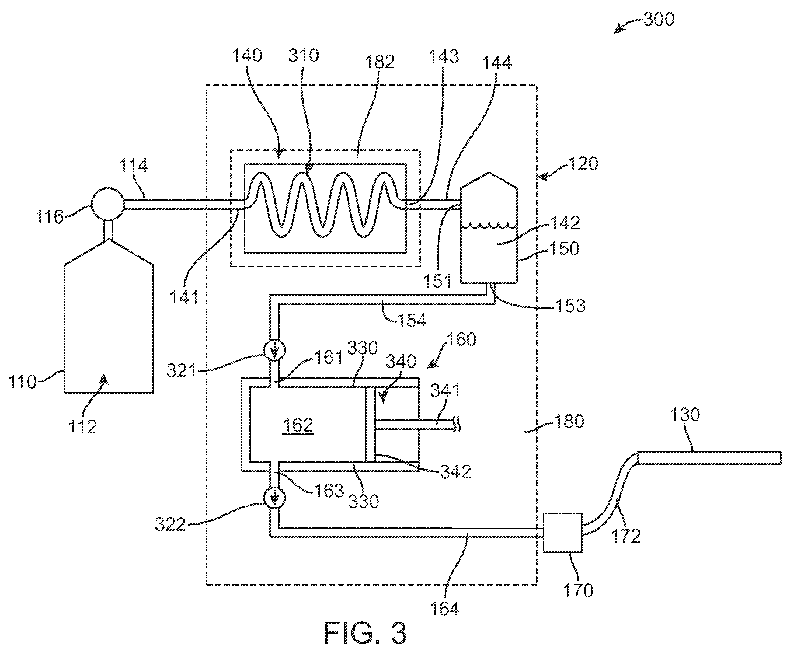

Fig. 3 illustrates one embodiment of a system 300 that is generally configured as shown in Figs. 1 and 2, and in which the cooling element 140 is a heat exchanger 310. The cooling element 140 may also be other devices or components other than a heat exchanger assuming they can cool gaseous coolant 112 to sufficiently low temperatures to liquefy the gaseous coolant 112. A heat exchanger 310 is provided as one example of a cooling element 140 that can be used in embodiments. Valves, such as one-way check valves 321, 322, may be utilized to control the liquid coolant 142 that enters and exits the chamber 162.

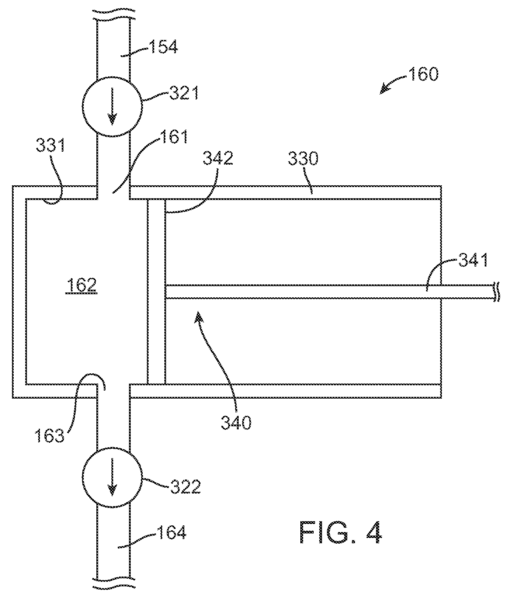

With further reference to Fig. 4, one embodiment of an actuator 160 is in the form of a pump, a piston or a syringe or like device that defines an internal chamber 162. For ease of explanation, reference is made to an actuator 160 that includes an outer body 330 having an inner surface 331 and that defines the inner chamber 162. A component 340 is movable within the interior space defined by the outer body 330 and can be controlled or manipulated, e.g., by another component of the control system 120 (such as a processor, computer, motor or other actuator) to push or pull the component 340 and adjust the size or volume of the chamber 162. A user may also be able to manually push or pull the component 340 depending on the liquid 142 pressure within the chamber 162. The component 340 includes a seal or gasket type device 342 that is movable within the outer body 330 by pushing or pulling a rod 341 or like component. The outer surface or perimeter of the seal 342 of the movable component 340 interfaces with and forms a seal with the inner surface 331 of the actuator 160 so that liquid 142 is retained inside the chamber 162. The pressure inside the chamber 162 may range from about 10 psi vacuum to about 1,600 psi.

The actuator 160 is configured to controllably draw liquid coolant 142 liquid into the chamber 162, and to controllably expel or force liquid coolant 142 out of the chamber 162 for delivery to the cryo-ablation catheter 130. For this purpose, the input valve 321 may be a one-way valve such as a check valve so that the liquid coolant 142 can be drawn from the container 140 and into the chamber 162, but cannot flow in the opposite direction from the chamber 162 and back into the container 140. Similarly, the output valve 322 may be a one-way valve such as a check valve so that liquid coolant 142 can be forced out of the chamber 162 through the output valve 322, but not back through the input valve 321.

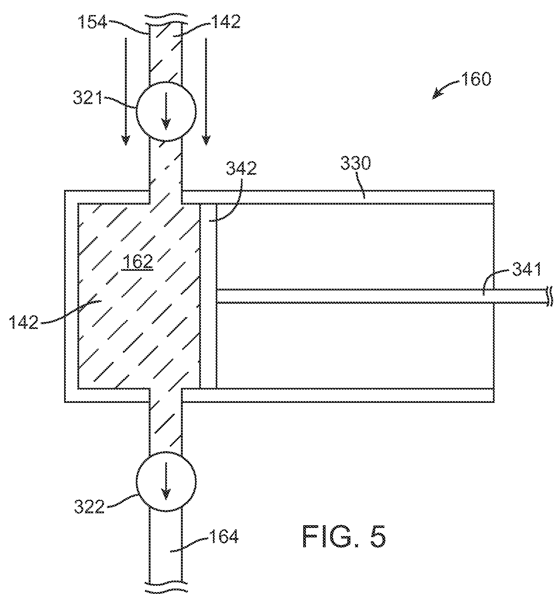

According to one embodiment, liquid coolant 142 is permitted to flow through the one-way inlet valve 321 if a sufficiently high pressure difference or cracking pressure exists between the supply line 154 and/or container 140 and the chamber 162. Similarly, according to one embodiment, liquid coolant 142 is permitted to flow through the one-way outlet valve 322 if a sufficient high pressure difference or cracking pressure exists between the chamber 162 and the supply line 164 and/or cryo-ablation catheter 130. Figs. 5-7 further illustrate how the actuator 160 is utilized to selectively and controllably deliver liquid coolant 142 to a cryo-ablation device 130.

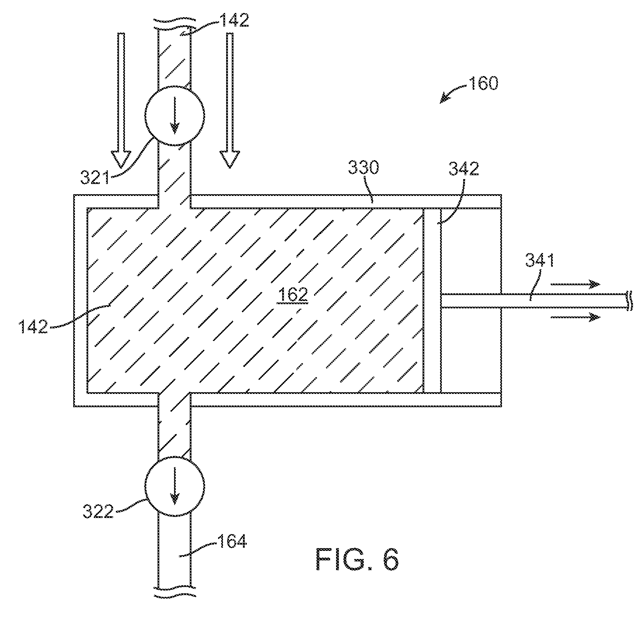

Referring to Fig. 5, in one embodiment, liquid coolant 142 from the container 140 may flow or be controllably drawn through the input valve 321 and into the chamber 162 (represented by arrows) as the movable component 340 is initially pulled backwards. This motion results in a reduction of pressure within the chamber 162 and a pressure differential between the supply line 154 / container 140 and the chamber 162 that is greater than the cracking pressure of the one-way inlet valve 321. As a result, liquid coolant 142 is maintained within the chamber 162, and does not flow out of the chamber 162 through the outlet valve 322 due to the suction-type action resulting from pulling the component 342 back to enlarge the chamber 162. Fig. 6 illustrates the movable component 140 being pulled backwards to a greater degree such that the chamber 162 volume increases, thereby controllably loading or drawing more liquid coolant 142 into the chamber 162.

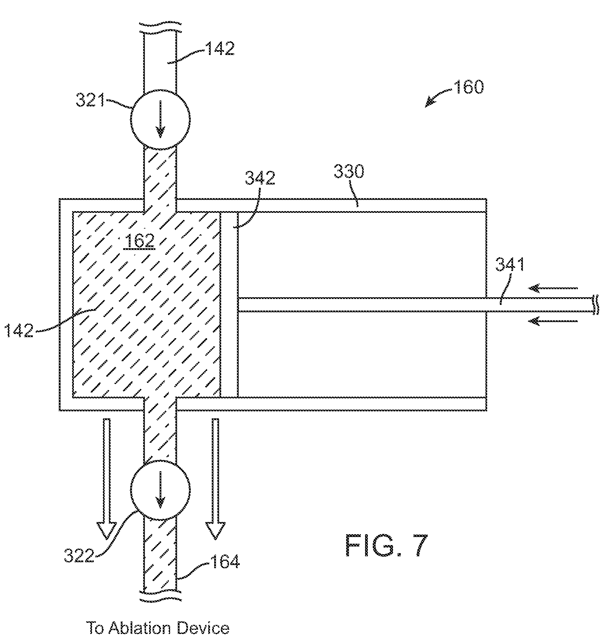

Referring to Fig. 7, when it is decided or determined that liquid coolant 142 is to be delivered to the cryo-ablation catheter 130, the component 140 is pushed in a forward direction which, in turn, reduces the size or volume of the chamber 162 and pushing, forcing or expelling the liquid coolant 142 out of the chamber 162 through the outlet valve 322. Since both valves 321, 322 are one-way valves, liquid coolant 142 only flows out of the chamber 162 through the outlet valve 322 (represented by arrows), whereas the inlet valve 321 prevents liquid coolant 142 from flowing back into the supply line 154 and container 140. The pressure of the liquid coolant 142 expelled by from the chamber 162 can be set or adjusted based on one or more or all of the cracking pressure of the outlet valve 322, the speed at which the component 140 is moved, dimensions of the chamber 162 and dimensions of line 164.

Thus, embodiments are configured to provide a storage container 140 and an actuator 160 that are positioned between the output 142 of the cooling element 140 and a cryo-ablation catheter 130 or interface 170 thereto. In this manner, embodiments provide "indirect" injection of liquid coolant 142 to the cryo-ablation catheter 130 and advantageously allow liquid coolant 142 to be selectively and controllably delivered to a cryo-ablation catheter as needed and at desired pressures.

For example, liquid coolant 142 can be stored in the container 150, and when a user needs the liquid coolant 142, the liquid coolant 142 can be controllably loaded into the chamber 162. The user can then decide when to deliver the liquid coolant 142 from the chamber 162 to the cryo-ablation catheter 130. Embodiments, therefore, advantageously allow a user or another component of the control system 120 (such as a processor, computer, motor or other actuator) to determine and control the timing of when liquid coolant 142 is delivered to the cryo-ablation catheter 130. Additionally, embodiments advantageously allow a user or another component of the control system 120 to deliver liquid coolant 132 continuously, periodically, intermittently, or not at all.

For example, after liquid coolant 142 is loaded into the chamber 162, the movable component 140 can be pulled back to expand the chamber 162 and controllably load or draw in liquid coolant 142 from the container and into the chamber 162, and then be pushed so that all or a substantial portion of the liquid coolant 142 is forced out of the chamber 162, e.g., in a continuous manner, to continuously deliver liquid coolant 142 to the cryo-ablation catheter 130. The movable component 140 may be moved at a constant rate of speed so that liquid coolant 142 is delivered to the cryo-ablation catheter 130 at a constant flow rate. In an alternative embodiment, the movable component 140 can be pushed at different speeds if it is desirable to deliver more liquid coolant 142 at certain times and less liquid coolant 142 at other times. As a further alternative embodiment, pushing or movement of the component 140 can be interrupted to stop or reduce the flow of liquid coolant 142 to the cryo-ablation catheter 130. Thus, embodiments advantageously allow a user or other component of the control system 120 to control and adjust the quantity, timing and rate at which liquid coolant 142 is delivered to the cryo-ablation catheter 130.

In addition to these enhanced controls, embodiments also advantageously allow the liquid coolant 142 to be delivered at various pressures including pressures that exceed the pressure inside the supply tank 110. More specifically, since the actuator 160 is independent of the supply tank 110, the pressure of the liquid coolant 142 in the chamber 162 is also independent of the pressure of the gaseous coolant 112 and the pressure of the liquid coolant 142 output by the cooling element 140. In this manner, the pressure of the liquid coolant 142 can be set or selected to be at a desired pressure that may be less than or greater than the pressure of the gaseous coolant 112 inside the supply tank 110 and the pressure of the liquid coolant 142 output by the cooling element 140.

Liquid coolant 142 pressures that are greater than pressures achieved based on the supply tank 110 are advantageous to ensure that the liquid coolant 142 that is delivered to the cryo-ablation catheter 130 remains a liquid at acceptable pressures and temperatures. More specifically, after the liquid coolant 142 is pushed out of the chamber 162 and flows through the supply line 164, the temperature of the liquid coolant 142 may increase (e.g., due to heat generated by friction or the surrounding environment while the liquid coolant 142 flows through the supply line 164). Depending on the type of liquid coolant 142 that is utilized, temperature differences may cause the liquid coolant to change form, e.g., to become vapor or a gas, in which case the cryo-ablation catheter 130 that is configured for use with a liquid will not function properly or will be less effective. In known systems, liquid coolant 142 pressures that are based on or limited by the pressure of the supply tank 110 may not be sufficient to maintain the liquid coolant 142 as a liquid as the liquid passes through the supply line 164 and/or 172 and is heated. However, embodiments advantageously allow liquid coolant 142 to flow through the supply lines 164 and/or 172 to the cryo-ablation catheter 130 at pressure levels that are higher than pressures levels of the supply tank 110, thereby allowing the liquid coolant 142 to be maintained as a liquid even if the temperature of the liquid coolant 142 is increased. Referring again to Figs. 1 and 3, a substantial portion of the supply lines 164 and/or 172 can be maintained within a cooled environment 180 in order to reduce the effects of elevated temperatures on the liquid coolant 142. Further, the length of the remaining supply line 172 that connects to the cryo-ablation catheter 130 can be reduced or minimized while still permitting use and manipulation of the cryo-ablation catheter 130.

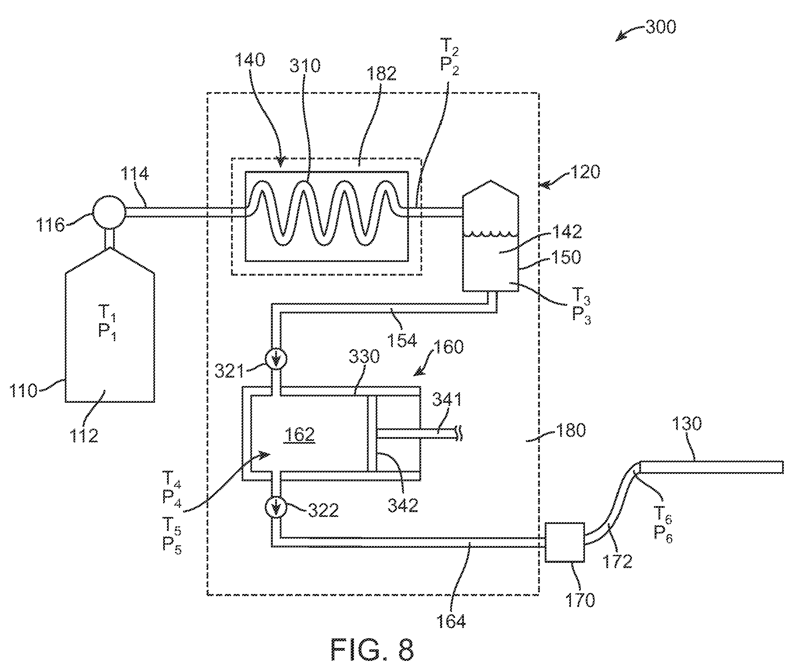

Fig. 8 illustrates examples of operating parameters of one example of a control system 820 that utilizes liquid nitrous oxide 142 that is delivered to a cryo-ablation catheter 130. In the illustrated embodiment, the supply tank 110 includes gaseous nitrous oxide 112 at a pressure P1 of about 10.3 Bar (150 psi) to about 55.2 Bar (800 psi), e.g., about 48.3-55.2 Bar (700-800 psi) and a temperature T1 of about 10°C. to about 25°C., e.g., about 15°. The gaseous nitrous oxide 112 is provided to a cooling element 140, and the resulting liquid nitrous oxide 142 is at a pressure P2 of about 13.8 Bar to about 55.2 Bar (200 psi to about 800 psi), e.g., 48.3-55.2 Bar (700-800 psi) (based on the pressure of the supply tank 110) and a temperature T2 of about -70°C. to about 0°C., e.g., about -30°C. to about -40°C. The liquid nitrous oxide 142 is then stored in the container 150 at similar pressures P3, e.g., about 48.3-55.2 Bar (700-800 psi) and similar temperatures T3, e.g., about -30°C. to about -40°C. The cracking pressure of the input valve 321 may be about 0.7 Bar to about 3.4 Bar (10 psi to about 50 psi). The pressure P4 of the liquid nitrous oxide 142 within the chamber 162 (e.g., as shown in Fig. 6) when the chamber 162 is enlarged and a maximum amount of liquid nitrous oxide 142 is drawn into the chamber 162 may be about 10.3 Bar to about 68.9 Bar (150 psi to about 1000 psi), e.g., about 41.4 Bar (600 psi), and the temperature T4 of the liquid nitrous oxide 142 may be about -70°C. to about 0°C, e.g., about -40°C. to about -30°C. The pressure P5 of the liquid nitrous oxide 142 within the chamber 162 (e.g., as shown in Fig. 7) when the size of the chamber 162 is reduced or minimized and fluid nitrous oxide 142 is pushed out of the chamber 162) may be about 3.4 Bar to about 5.5 Bar (50 psi to about 80 psi), e.g., about 55.2 Bar (800 psi), and the temperature T5 of the liquid nitrous oxide 142 may be about-70°C. to about 0°C, e.g., about -40°C. to about -30°C. As the liquid nitrous oxide 142 flows through the supply lines, the temperature of the fluid 142 may increase. For example, the pressure P6 of the liquid nitrous oxide 142 delivered to the cryo-ablation catheter 130 may be about 10.3 Bar to about 68.9 Bar (150 psi to about 1000 psi), e.g., about 55.2 Bar (800 psi), and the temperature T6 of the liquid nitrous oxide 142 delivered to the cryo-ablation catheter 130 may be about -70°C. to about 0°C, e.g., about -40°C. to about -30°C.

Since embodiments are able to achieve higher pressures compared to known "direct injection" systems, embodiments are advantageously capable of maintaining nitrous oxide in liquid form even at elevated temperatures that would normally result in nitrous oxide gas or vapor. Additionally, as higher pressures are needed, the actuator 160 can be configured to increase the pressure to even higher levels, e.g., by moving the component 140 more quickly. Further, if additional liquid nitrous oxide 142 is needed, the component 140 can be pulled back to load additional liquid, and then be pushed to force the additional liquid out of the chamber 162 and into the supply line 142 to the cryo-ablation catheter 130. This process can be repeated as necessary to deliver desired quantities of liquid nitrous oxide 142 to the cryo-ablation catheter 130.

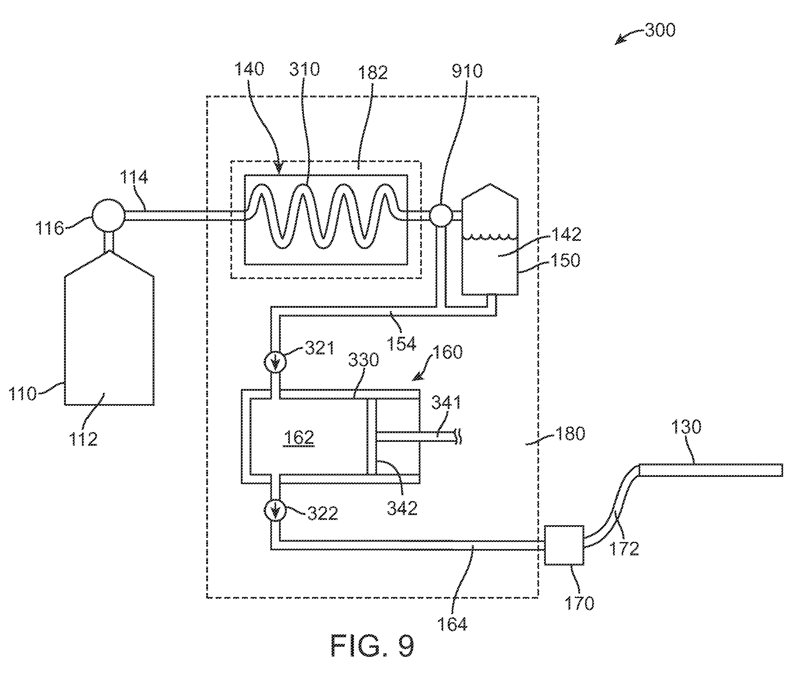

Referring to Fig. 9, in an alternative embodiment, a system 900 for controllably delivering liquid coolant 142 to a cryo-ablation catheter 130 is similar to the system 100 shown in Fig. 1 in that liquid coolant 142 is not injected directly into a cryo-ablation catheter 130 and instead is controllably delivered using an actuator 160. The system 900 also includes a bypass valve 910 that connected between the cooling element 140 and the container 150. With this configuration, the bypass valve 910 can be controlled to selectively store liquid coolant 142 in the container 150 and provide liquid coolant 142 to the actuator 160 as needed, or to provide liquid coolant 142 output by the cooling element 140 to the actuator 160 without storing the liquid coolant 142. For example, the supply line 154 may be filled with liquid coolant 142 until the liquid coolant 142 is drawn into the chamber 162 when component 140 is stationary and not pulled back. Liquid coolant 142 may then be controllably drawn into the chamber 162 from the cooling element 140, and may subsequently be controllably expelled or forced from the chamber 162 when the movable component 140 is pushed to reduce the size of the chamber 162.

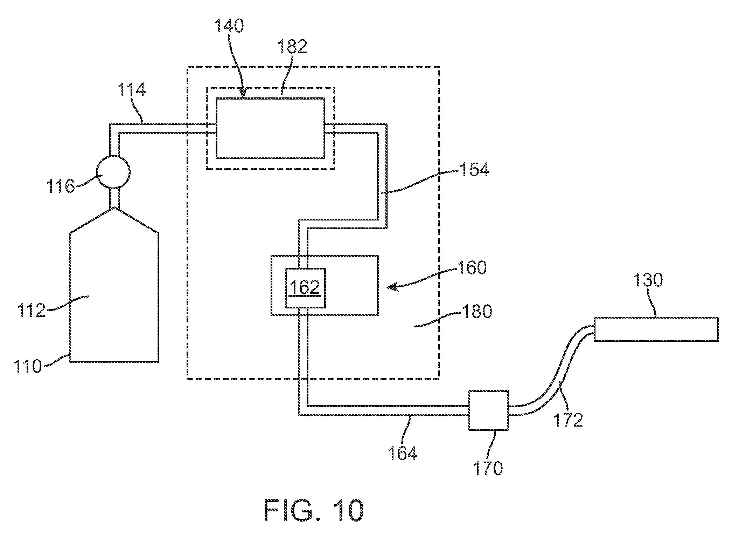

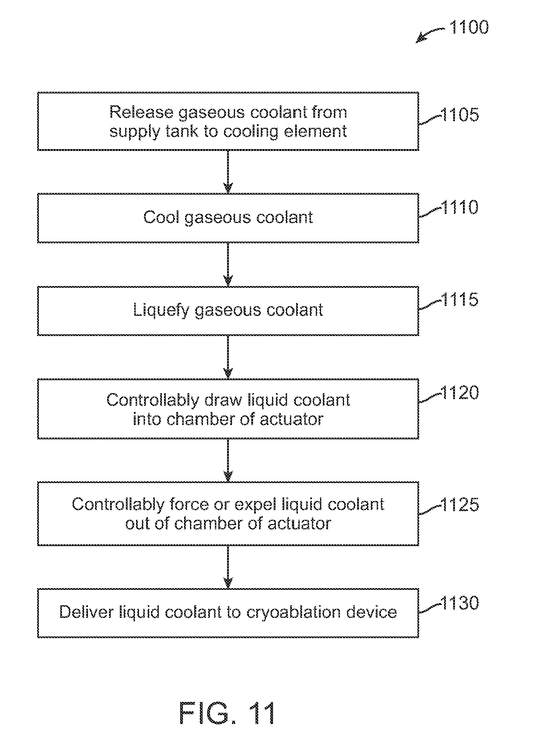

Referring to Fig. 10, and with further reference to Fig. 11, a system 1000 for controllably delivering liquid coolant 142 to a cryo-ablation catheter 130 includes no container 150 and no bypass valve 910. Thus, the system 1000 shown in Fig. 10 includes the components shown in Fig. 1 except for the container 150, and includes the components shown in Fig. 9 except for the bypass valve 910 and the container 150. The system 1000 may use operating parameters that are the same as or similar to parameters described with reference to Fig. 8.

With the system 1000 configuration shown in Fig. 10, and with the configuration shown in Fig. 9 in which the container 150 is bypassed by the valve 910, a method 1100 of delivering liquid coolant 142 to a cryo-ablation device 130 includes releasing gaseous coolant 112 from the supply tank 110 to the cooling element 140 at stage 1105, e.g., by opening or controlling a valve 116. At stage 1110, the gaseous coolant 112 is cooled, and at stage 1115, the gaseous coolant condenses into a liquid 142 and flows into a supply line 154 until it flows or is drawn into the chamber 162 of the actuator 160 at stage 1120. At stage 1125, the liquid coolant 142 is controllably forced or expelled out of the chamber 162 and delivered to the cryo-ablation catheter 130 at stage 1130.

Since embodiments are able to achieve higher pressures compared to known "direct injection" systems, embodiments are advantageously capable of maintaining nitrous oxide in liquid form even at higher temperatures that would normally result in nitrous oxide gas or vapor. Additionally, if higher pressures are needed, the actuator 160 can be so configured to increase the pressure to even higher levels, e.g., by moving the component 140 more quickly. Further, if additional liquid nitrous oxide is needed, the component 140 can be pulled back to load additional liquid nitrous oxide, and then be pushed to force the additional liquid nitrous oxide out of the chamber 162 and into the supply line 164 to the cryo-ablation catheter 130. This process can be repeated as necessary to deliver the necessary quantities of liquid nitrous oxide 142 for the cryo-ablation procedure.

For example, although embodiments are described with reference to a cooling element generating a liquid coolant, the cooling element may be deactivated so that gaseous coolant passes through the cooling element and can be processed by or delivered to other downstream components. Further, certain indirect injection systems may include a storage container, whereas others do not. Moreover, although examples of temperatures and pressures have been provided, it should be understood that other temperatures and pressures can be utilized, and the particular operating parameters can depend on the particular system configuration and type of gaseous and liquid coolants that are utilized.

## Claims

1. A system (100, 300) for delivering a liquid coolant (142) to a cryo-ablation device (130) configured for cryogenically ablating tissue, the system (100, 300) comprising:
a supply (110) of gaseous coolant (112); and
a cooling element (140) configured to liquefy gaseous coolant (112) released from the supply (110);
**characterized in that** the system (100, 300) further comprises a container (150) configured to collect or store liquid coolant (142) generated by the cooling element (140), and an actuator (160) in fluid communication with the cooling element (140) and the cryo-ablation device (130), the actuator (160) defining a chamber (162) and being configured to controllably draw liquid coolant (142) into the chamber (162) from the container (150), which is connected between the cooling element (140) and the actuator (160), the actuator further configured to controllably expel liquid coolant (142) from the chamber (162) for delivery to the cryo-ablation device (130).

2. The system (100, 300) of claim 1, further comprising:
a first one-way valve (321) associated with an input (161) of the actuator (160), and
a second one-way valve (322) associated with an output (163) of the actuator (160), the first and second one-way valves (321, 322) controlling flow of liquid coolant (142) into and out of the chamber (162), respectively.

3. The system (100, 300) of claim 1, wherein the actuator (160) is a syringe comprising:
a hollow barrel defining the chamber (162),
a gasket (342) disposed within the hollow barrel, and
a plunger that is controllably movable to displace the gasket (342) and to expand the chamber (162) to controllably draw liquid coolant (142) into the chamber (162), and to contract the chamber (162) to controllably expel liquid coolant (122) from the chamber (162).

4. The system (100, 300) of claim 1, wherein the actuator (160) is a pump.

5. The system (100, 300) of claim 1, the actuator (160) including a piston that is controllably moveable within a hollow body to expand the chamber (162) and controllably draw liquid coolant (142) into the chamber (162), and to contract the chamber (162) and controllably expel liquid coolant (142) from the chamber (162).

6. The system (100, 300) of claim 1, wherein the gaseous coolant (112) is nitrous oxide.

7. The system (100, 300) of claim 1, wherein the cryo-ablation device (130) is a cryo-ablation catheter.

8. The system (100, 300) of claim 1, the actuator (160) being configured such that a pressure of liquid coolant (142) within the chamber (162) is greater than a pressure of gaseous coolant (112) within the supply (110).

9. The system (100, 300) of claim 1, wherein the cooling element (140) is a heat exchanger (310).

10. The system (100, 300) of claim 1, the cooling element (140) and the actuator (160) being contained within a common cooling environment.

11. The system (100, 300) of claim 10, the cooling element (140) being contained within a separate cooling environment within the common cooling environment.

## Patentansprüche

1. System (100, 300) zum Zuführen eines flüssigen Kühlmittels (142) zu einer Cryoablationsvorrichtung (130), die zum cryogenen Abtragen von Gewebe konfiguriert ist, welches System (100, 300) aufweist:
eine Zuführung (110) für gasförmiges Kühlmittel (112); und
ein Kühlelement (140), das zum Verflüssigen des von der Zuführung (110) freigegebenen gasförmigen Kühlmittels (112) konfiguriert ist;
**dadurch gekennzeichnet, dass** das System (100, 300) weiterhin aufweist: einen Behälter (150), der konfiguriert ist zum Sammeln oder Speichern von von dem Kühlelement (140) erzeugtem flüssigem Kühlmittel (142), und
einen Aktuator (160) in Fluidverbindung mit dem Kühlelement (140) und der Cryoablationsvorrichtung (130), wobei der Aktuator (160) eine Kammer (162) definiert und konfiguriert ist zum steuerbaren Ziehen von flüssigem Kühlmittel (142) aus dem Behälter (150), der zwischen dem Kühlelement (140) und dem Aktuator (160) angeordnet ist, in die Kammer (162), und der Aktuator weiterhin konfiguriert ist zum steuerbaren Ausstoßen von flüssigem Kühlmittel (142) aus der Kammer (162) für die Zuführung zu der Cryoablationsvorrichtung (130).

2. System (100, 300) nach Anspruch 1, weiterhin aufweisend:
ein erstes Einweg-Ventil (321), das mit einem Eingang (161) des Aktuators (160) assoziiert ist, und
ein zweites Einweg-Ventil (322), das mit einem Ausgang (163) des Aktuators (160) assoziiert ist,
wobei das erste und das zweite Einweg-Ventil (321, 322) die Strömung von flüssigem Kühlmittel (142) in die bzw. aus der Kammer (162) steuern.

3. System (100, 300) nach Anspruch 1, bei dem der Aktuator (160) eine Spritze ist, welche aufweist:
einen hohlen Zylinder, der die Kammer (162) definiert,
eine Dichtung (342), die in dem hohlen Zylinder angeordnet ist, und
einen Druckkolben, der steuerbar bewegbar ist, um die Dichtung (342) zu verschieben und die Kammer (162) zu erweitern zum steuerbaren Ziehen von flüssigem Kühlmittel (142) in die Kammer (162), und die Kammer (162) zusammenzuziehen zum steuerbaren Ausstoßen von flüssigem Kühlmittel (122) aus der Kammer (162).

4. System (100, 300) nach Anspruch 1, bei dem der Aktuator (160) eine Pumpe ist.

5. System (100, 300) nach Anspruch 1, bei dem der Aktuator (160) einen Kolben enthält, der steuerbar innerhalb eines hohlen Körpers bewegbar ist, um die Kammer (162) zu erweitern und steuerbar flüssiges Kühlmittel (142) in die Kammer (162) zu ziehen, und um die Kammer (162) zusammenzuziehen und steuerbar flüssiges Kühlmittel (142) aus der Kammer (162) auszustoßen.

6. System (100, 300) nach Anspruch 1, bei dem das gasförmige Kühlmittel (112) Stickstoffoxid ist.

7. System (100, 300) nach Anspruch 1, bei dem die Cryoablationsvorrichtung (130) ein Cryoablationskatheter ist.

8. System (100, 300) nach Anspruch 1, bei dem der Aktuator (160) so konfiguriert ist, dass ein Druck von flüssigem Kühlmittel (142) innerhalb der Kammer (162) größer als ein Druck von gasförmigem Kühlmittel (112) innerhalb der Zuführung (110) ist.

9. System (100, 300) nach Anspruch 1, bei dem das Kühlelement (140) ein Wärmetauscher (310) ist.

10. System (100, 300) nach Anspruch 1, bei dem das Kühlelement (140) und der Aktuator (160) innerhalb eines gemeinsamen Kühlumfelds enthalten sind.

11. System (100, 300) nach Anspruch 10, bei dem das Kühlelement (140) innerhalb eines separaten Kühlumfelds innerhalb des gemeinsamen Kühlumfelds enthalten ist.

## Revendications

1. Système (100, 300) pour délivrer un réfrigérant liquide (142) à un dispositif de cryo-ablation (130) configuré pour réaliser l'ablation cryogénique d'un tissu, le système (100, 300) comprenant :
un moyen d'alimentation (110) de réfrigérant gazeux (112) ; et
un élément de réfrigération (140) configuré pour liquéfier du réfrigérant gazeux (112) libéré depuis le moyen d'alimentation (110),
**caractérisé en ce que** le système (100, 300) comprend en outre un conteneur (150) configuré pour collecter ou stocker du réfrigérant liquide (142) généré par l'élément de réfrigération (140) et un actionneur (160) en communication de fluide avec l'élément de réfrigération (140) et le dispositif de cryo-ablation (130), l'actionneur (160) définissant une chambre (162) et étant configuré pour entraîner de façon contrôlable du réfrigérant liquide (142) à l'intérieur de la chambre (162) depuis le conteneur (150), lequel est connecté entre l'élément de réfrigération (140) et l'actionneur (160), l'actionneur étant en outre configuré pour expulser de façon contrôlable du réfrigérant liquide (142) depuis la chambre (162) pour sa délivrance au dispositif de cryo-ablation (130).

2. Système (100, 300) selon la revendication 1, comprenant en outre :
une première valve une voie (321) associée à une entrée (161) de l'actionneur (160) ; et
une seconde valve une voie (322) associée à une sortie (163) de l'actionneur (160), les première et seconde valves une voie (321, 322) contrôlant l'écoulement du réfrigérant liquide (142) respectivement à l'intérieur de la chambre (162) et à l'extérieur de celle-ci.

3. Système (100, 300) selon la revendication 1, dans lequel l'actionneur (160) est une seringue comprenant :
un réservoir creux définissant la chambre (162) ;
un joint d'étanchéité (342) disposé à l'intérieur du réservoir creux ; et
un plongeur qui peut être rendu mobile de façon contrôlable de manière à déplacer le joint d'étanchéité (342) et à dilater la chambre (162) afin d'entraîner de façon contrôlable du réfrigérant liquide (142) à l'intérieur de la chambre (162) et de manière à contracter la chambre (162) afin d'expulser de façon contrôlable du réfrigérant liquide (122) depuis la chambre (162).

4. Système (100, 300) selon la revendication 1, dans lequel l'actionneur (16) est une pompe.

5. Système (100, 300) selon la revendication 1, l'actionneur (160) incluant un piston qui peut être rendu mobile de façon contrôlable à l'intérieur d'un corps creux de manière à dilater la chambre (162) et à entraîner de façon contrôlable du réfrigérant liquide (142) à l'intérieur de la chambre (162) et de manière à contracter la chambre (162) et à expulser de façon contrôlable du réfrigérant liquide (142) depuis la chambre (162).

6. Système (100, 300) selon la revendication 1, dans lequel le réfrigérant gazeux (112) est de l'oxyde nitreux.

7. Système (100, 300) selon la revendication 1, dans lequel le dispositif de cryo-ablation (130) est un cathéter de cryo-ablation.

8. Système (100, 300) selon la revendication 1, l'actionneur (160) étant configuré de telle sorte qu'une pression du réfrigérant liquide (142) à l'intérieur de la chambre (162) soit supérieure à une pression du réfrigérant gazeux (112) à l'intérieur du moyen d'alimentation (110).

9. Système (100, 300) selon la revendication 1, dans lequel l'élément de réfrigération (140) est un échangeur thermique (310).

10. Système (100, 300) selon la revendication 1, l'élément de réfrigération (140) et l'actionneur (160) étant contenus à l'intérieur d'un environnement de réfrigération commun.

11. Système (100, 300) selon la revendication 10, l'élément de réfrigération (140) étant contenu à l'intérieur d'un environnement de réfrigération séparé à l'intérieur de l'environnement de réfrigération commun.
